# EUROPEAN PATENT SPECIFICATION

(11) **EP 1 737 450 B1**
(45) Date of publication and mention of the grant of the patent: **16.09.2009**
(21) Application number: 05721534.5
(22) Date of filing: 18.03.2005
(51) Int. Cl.: A61K 31/426, C07D 277/38, C07D 417/06

(54) **AQUEOUS COMPOSITION COMPRISING A THIAZOLE DERIVATIVE**
WÄSSRIGE ZUSAMMENSETZUNG MIT EINEM THIAZOL-DERIVAT
COMPOSITION AQUEUSE COMPRENANT UN DÉRIVÉ DE THIAZOLE

(30) Priority: 18.03.2004 US 553956 P
(43) Date of publication of application: 03.01.2007
(73) Proprietor: R-Tech Ueno, Ltd., Tokyo 1000011 (JP); Astellas Pharma Inc., Tokyo 103-8411 (JP)
(72) Inventor: UENO, Ryuji, Montgomery, Maryland 20854 (US); HIRATA, Ryu, 6691324 (JP); HARADA, Yasuhiro, 6691321 (JP)
(74) Representative: Albrecht, Thomas
(86) International application number: PCT/JP2005/005607
(87) International publication number: WO 2005/089755

(56) References cited:
- US-A- 4 780 465
- US-A1- 2004 259 923

## Description

### TECHNICAL FIELD

The present invention relates to an aqueous composition comprising a specific thiazole derivative.

### BACKGROUND ART

Vascular adhesion protein-1 (hereinafter to be abbreviated as VAP-1) is an amine oxidase (semicarbazide sensitive amine oxidase, SSAO) which is abundant in human plasma, and shows remarkably increased expression in vascular endothelium and vascular smooth muscle of the' inflammatory region. While the physiological role of VAP-1 has not been clarified until recently, VAP-1 gene was cloned in 1998, and VAP-1 has been reported to be a membrane protein that regulates rolling and migration of lymphocyte and NK cell as an adhesion molecule under regulation of expression by inflammatory cytokine. Although the amine to be a substrate is unknown, it is considered to be methylamine generated in any part of living organisms. It is also known that hydrogen peroxide and aldehydes produced due to the amine oxidase activity in the molecule are important factors of adhesion activity.

Thiazole derivatives represented by formula (A) below are useful as VAP-1 inhibitor (US Patent Publication No. 20040259923A1 published on December 23, 2004.

R¹-NH-X-Y-Z (A)

wherein
R¹ is acyl;
X is a bivalent residue derived from optionally substituted thiazole;
Y is a bond, lower alkylene, lower alkenylene or -CONH-; and
Z is a group of the formula: 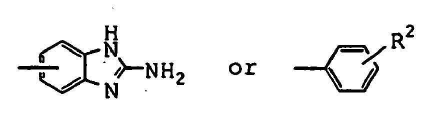 wherein R² is a group of the formula: -A-B-D-E
wherein A is a bond, lower alkylene, -NH- or -SO₂-;
B is a bond, lower alkylene, -CO- or -O-;
D is a bond, lower alkylene, -NH- or -CH₂NH-; and
E is optionally protected amino, -N=CH₂, 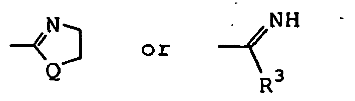 wherein
Q is -S- or -NH-; and
R³ is hydrogen, lower alkyl, lower alkylthio or -NH-R⁴ wherein R⁴ is hydrogen, -NH₂ or lower alkyl;
or a pharmaceutically acceptable salt thereof.

Furthermore, document US 4,780,465 discloses a stable isotonic aqueous solution consisting essentially of an effective microbiocidal amount of 1-ethyl-6,8-difluoro-1,4-dihydro-7-(3-methyl-1-piperazinyl)-4-oxoquinoline-3-carboxylic acid or a pharmaceutically acceptable salt thereof and an isotonizing amount of a compound selected from the group consisting of a polyalcohol, selected from the group consisting of glycerin, mannitol, glucose, xylitol, xylose, sorbitol and propylene glycol, and boric acid as an isotonizing agent for the solution, the solution being adjusted to a pH of about 3 to 6.5.

Usually, in preparing an aqueous dosage forms such as injection, eye drops and the like, sodium chloride is added to adjust osmotic pressure of the solution. However, in preparing an aqueous composition comprising the above thiazole derivative, there is a problem that the presence of sodium chloride in the solution decreases the solubility of the above thiazole derivative and precipitates the derivative.

### SUMMARY OF THE INVENTION

An object of the present invention is to provide an aqueous composition comprising a certain thiazole derivative which is clear and stable, and can be stored for a long time.

Thus, the present invention provides
[1] An aqueous composition comprising a compound of formula (I) [hereinafter sometimes referred to as Compound (I)]:

   R¹-NH-X-Y-Z (I)

   wherein
   R¹ is acyl;
   X is a bivalent residue derived from optionally substituted thiazole;
   Y is a bond, lower alkylene, lower alkenylene or -CONH-; and
   Z is a group of the formula: 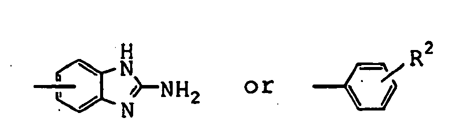 wherein R² is a group of the formula: -A-B-D-E
   wherein A is a bond, lower alkylene, -NH- or -SO₂-;
   B is a bond, lower alkylene, -CO- or -O-;
   D is a bond, lower alkylene, -NH- or -CH₂NH-, provided that when B is -CO- or -O-, D is not a bond; and
   E is optionally protected amino, -N=CH₂, 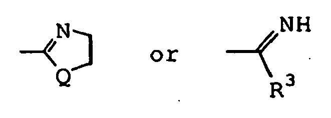 wherein Q is -S- or -NH-; and
   R³ is hydrogen, lower alkyl, lower alkylthio or -NH-R⁴
   wherein R⁴ is hydrogen, -NH₂ or lower alkyl; or a pharmaceutically acceptable salt thereof, water and an additive selected from the group consisting of polyol, sugar other than glucose, sugar alcohol, boric acid or its salt.
[2] The composition of [1], wherein Z of the compound (I) is a group of the formula: 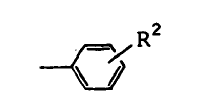 wherein R² is a group of the formula: 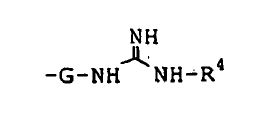 (wherein G is a bond, -NHCOCH₂- or lower alkylene and R⁴ is hydrogen, -NH₂ or lower alkyl); -NH₂; -CH₂NH₂; -CH₂ONH₂;
   -CH₂ON=CH₂; 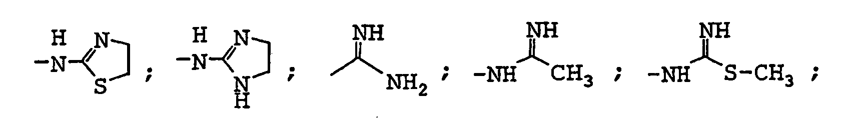 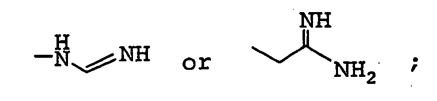 or a pharmaceutically acceptable salt thereof.
[3] The composition of [2], wherein R² of the compound (I) is a group of the formula: 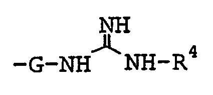 (wherein G is a bond, -NHCOCH₂- or lower alkylene and R⁴ is hydrogen or lower alkyl) ; -CH₂NH₂; -CH₂ONH₂; -CH₂ON=CH₂; 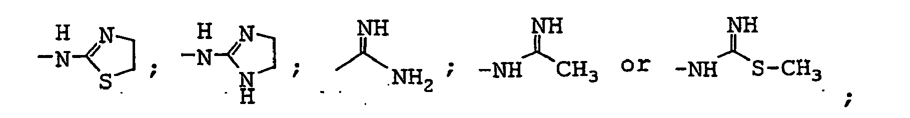 or a pharmaceutically acceptable salt thereof.
[4] The composition of any of [1] to [3], wherein R¹ of the compound (I) is alkylcarbonyl and X is a bivalent residue derived from thiazole optionally substituted by methylsulfonylbenzyl, or a pharmaceutically acceptable salt thereof.
[5] The composition of [1], wherein the compound (I) is N-{4-[2-(4-{[amino(imino)methyl]amino}phenyl)ethyl]-1,3-thiazol-2-yl}acetamide,
   N-{4-[2-(4-{[amino(imino)methyl]amino}phenyl)ethyl]-5-[4-(methylsulfonyl)benzyl]-1,3-thiazol-2-yl}acetamide,
   N-{4-[2-(4-{[hydrazino(imino)methyl]amino}phenyl)ethyl]-5-[4-(methylsulfonyl)benzyl]-1,3-thiazol-2-yl}acetamide,
   N-{4-[2-(4-{[hydrazino(imino)methyl]amino}phenyl)ethyl]-1,3-thiazol-2-yl}acetamide, or
   N-(4-{2-[4-(2-{[amino(imino)methyl]amino}ethyl)phenyl] ethyl}-1;3-thiazol-2-yl)acetamide,
   or a pharmaceutically acceptable salt thereof.

### DETAILED DESCRIPTION OF THE INVENTION

In the above and subsequent descriptions of the present specification, suitable examples and illustration of the various definitions to be included within the scope of the invention are explained in detail as follows.
Suitable "halogen" includes fluorine, chlorine, bromine and iodine.

The term "lower" is used to intend a group having 1 to 6, preferably 1 to 4, carbon atom(s), unless otherwise provided.

Suitable "lower alkyl" includes straight or branched alkyl having 1 to 6 carbon atom(s), such as methyl, ethyl, propyl, isopropyl, butyl, isobutyl, sec-butyl, tert-butyl, pentyl, tert-pentyl and hexyl, in which more preferred one is C₁-C₄ alkyl.

Suitable "lower alkylthio" includes lower alkylthio containing the above lower alkyl, such as methylthio, ethylthio, propylthio, isopropylthio, butylthio, isobutylthio, sec-butylthio, tert-butylthio, pentylthio, tert-pentylthio and hexylthio.

Suitable "lower alkylene" includes straight or branched alkylene having 1 to 6 carbon atom(s), such as methylene, ethylene, trimethylene, tetramethylene, propylene, ethylidene and propylidene, in which more preferred one is C₁-C₄ alkylene.
Suitable "lower alkenylene" includes straight or branched alkenylene having 2 to 6 carbon atom(s), such as -CH=CH-, -CH₂-CH=CH-, -CH₂-CH=CH-CH₂-, -CH₂-CH₂-CH=CH-, -CH=CH-CH=CH-, -CH=CH-CH₂-CH₂-CH₂-, -CH=CH-CH=CH-CH₂-CH₂- and
-CH=CH-CH=CH-CH=CH-, in which more preferred one is C₂-C₄ alkenylene.

The above lower alkenylene may be in E or Z form, respectively. Thus, those skilled in the art will recognize that the lower alkenylene includes all E, Z-structures when it has 2 or more double bonds.
Suitable "aryl" includes C₆-C₁₀ aryl such as phenyl and naphthyl, in which more preferred one is phenyl. The "aryl" may be substituted by 1 to 3 substituent(s) and the substitution sites are not particularly limited.

Suitable "aralkyl" includes aralkyl wherein the aryl moiety has 6 to 10 carbon atoms [i.e. the aryl moiety is C₆-C₁₀ aryl of the above "aryl"] and the alkyl moiety has 1 to 6 carbon atom(s) [i.e. the alkyl moiety is C₁-C₆ alkyl of the above "lower alkyl"], such as benzyl, phenethyl, 1-naphthylmethyl, 2-naphthylmethyl, 3-phenylpropyl, 4-phenylbutyl and 5-phenylpentyl.

The "optionally protected amino" means that an amino group may be protected with a suitable protecting group according to a method known per se, such as the methods described in Protective Groups in Organic Synthesis, published by John Wiley and Sons (1980), and the like. The suitable "protecting group" includes tert-butoxycarbonyl (i.e., Boc), an acyl group as mentioned below, substituted or unsubstituted aryl(lower)alkylidene [e.g., benzylidene, hydroxybenzylidene, etc.], aryl(lower)alkyl such as mono-, di- or triphenyl-(lower)alkyl [e.g., benzyl, phenethyl, benzhydryl, trityl, etc.] and the like.

Suitable "optionally protected amino" includes amino and tert-butoxycarbonylamino (i.e. -NHBoc).

Suitable "heterocycle" includes "aromatic heterocycle" and "non-aromatic heterocycle".

Suitable "aromatic heterocycle" includes 5 to 10-membered aromatic heterocycle containing 1 to 3 heteroatom(s) selected from nitrogen, oxygen and sulfur atoms besides carbon atom(s), and includes, for example, thiophene, furan, pyrrole, imidazole, pyrazole, thiazole, isothiazole, oxazole, isoxazole, pyridine, pyridazine, pyrimidine, pyrazine and the like.

Suitable "non-aromatic heterocycle" includes 5 to 10-membered non-aromatic heterocycle containing 1 to 3 heteroatom(s) selected from nitrogen, oxygen and sulfur atoms besides carbon atom(s), and includes for example, pyrrolidine, imidazoline, pyrazolidine, pyrazoline, piperidine, piperazine, morpholine, thiomorpholine, dioxolan, oxazolidine, thiazolidine, triazolidine and the like.

Suitable "acyl" includes acyl having 1 to 20 carbon atom(s), such as formyl, alkylcarbonyl, arylcarbonyl, alkoxycarbonyl and aralkyloxycarbonyl.

Suitable "alkylcarbonyl" includes alkylcarbonyl wherein the alkyl moiety has 1 to 6 carbon atom (s) [i.e. the alkyl moiety is C₁-C₆ alkyl of the above "lower alkyl"], such as acetyl, propionyl, butyryl, isobutyryl, valeryl, isovaleryl, pivaloyl, hexanoyl and heptanoyl, in which more preferred one is C₁-C₄ alkyl-carbonyl.

Suitable "arylcarbonyl" includes arylcarbonyl wherein the aryl moiety has 6 to 10 carbon atom(s) [i.e. the aryl moiety is C₆-C₁₀ aryl of the above "aryl"], such as benzoyl and naphthoyl.

Suitable "alkoxycarbonyl" includes alkoxycarbonyl wherein the alkoxy moiety has 1 to 6 carbon atom(s), such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, tert-pentyloxycarbonyl and hexyloxycarbonyl, in which more preferred one is alkoxycarbonyl wherein the alkoxy moiety has 1 to 4 carbon atom(s).

Suitable "aralkyloxycarbonyl" includes aralkyloxycarbonyl wherein the aryl moiety has 6 to 10 carbon atom(s) [i.e. the aryl moiety is C₆-C₁₀ aryl of the above "aryl"] and the alkyl moiety has 1 to 6 carbon atom(s) [i.e. the alkyl moiety is C₁-C₆ alkyl of the above "lower alkyl"], such as benzyloxycarbonyl, phenethyloxycarbonyl, 1-naphthylmethyloxycarbonyl, 2-naphthylmethyloxycarbonyl, 3-phenylpropyloxycarbonyl, 4-phenylbutyloxycarbonyl and 5-phenylpentyloxycarbonyl.

Suitable "bivalent residue derived from thiazole" of the "bivalent residue derived from optionally substituted thiazole" includes

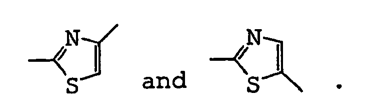

The "thiazole" may have 1 to 3 substituent(s) and the substitution sites are not particularly limited.

Suitable "substituent" of the above "optionally substituted thiazole" includes, for example,
(1) halogen which is as defined above;
(2) alkoxycarbonyl which is as defined above, such as ethoxycarbonyl;
(3) optionally substituted aryl, which aryl is as defined above and the substitution sites are not particularly limited, such as phenyl and 4-(methylsulfonyl)phenyl;
(4) a group of the formula: -CONR^{a}R^{b} wherein R^{a} is hydrogen, lower alkyl, aryl or aralkyl and R^{b} is hydrogen, lower alkyl, aryl or aralkyl, wherein the lower alkyl, aryl and aralkyl are as defined above, such as N-methylaminocarbonyl, N-phenylaminocarbonyl, N,N-dimethylaminocarbonyl and N-benzylasninocarbonyl;
(5) a group of the formula: -CONH-(CH₂)ₖ-aryl wherein k is an integer of 0 to 6; the aryl is as defined above, which may have 1 to 5 substituent(s) selected from the group consisting of -NO₂, -SO₂-(lower alkyl) wherein the lower alkyl is as defined above, -CF₃ and -O-aryl wherein the aryl is as defined above, and the substitution sites are not particularly limited;
(6) a group of the formula: -CONH-(CH₂)ₘ-heterocycle wherein m is an integer of 0 to 6; the heterocycle is as defined above, such as pyridine;
(7) a group of the formula: -CO-heterocycle wherein the heterocycle is as defined above, such as pyrrolidine, piperidine, piperazine, thiomorpholine, which may have 1 to 5 substituent(s) selected from the group consisting of -CO-(lower alkyl) wherein the lower alkyl is as defined above, -CO-O-(lower alkyl) wherein the lower alkyl is as defined above, -SO₂-(lower alkyl) wherein the lower alkyl is as defined above, oxo (i.e. =O) and a group of the formula: -CONR^{c}R^{d} wherein R^{c} is hydrogen, lower alkyl, aryl or aralkyl and R^{d} is hydrogen, lower alkyl, aryl or aralkyl wherein the lower alkyl, aryl and aralkyl are as defined above, and the substitution sites are not particularly limited;
(8) a group of the formula: -(CH₂)ₙ-aryl wherein n is an integer of 1 to 6; the aryl is as defined above, which may have 1 to 5 substituent(s) selected from the group consisting of -S-(lower alkyl) wherein the lower alkyl is as defined above, -SO₂-(lower alkyl) wherein the lower alkyl is as defined above, -CO₂-(lower alkyl) wherein the lower alkyl is as defined above, -NHCO-O-(lower alkyl) wherein the lower alkyl is as defined above and a group of the formula: -CONR^{e}R^{f} wherein R^{e} is hydrogen, lower alkyl, aryl or aralkyl and R^{f} is hydrogen, lower alkyl, aryl or aralkyl wherein the lower alkyl, aryl and aralkyl are as defined above, and the substitution sites are not particularly limited;
(9) a group of the formula: -(CH₂)ₒ-heterocycle wherein o is an integer of 0 to 6; the heterocycle is as defined above, such as pyrrolidine, piperidine, piperazine, morpholine, thiomorpholine, which may have 1 to 5 substituent(s) selected from the group consisting of oxo (i.e. =O); -CO- (lower alkyl) wherein the lower alkyl is as defined above; -CO-O-(lower alkyl) wherein the lower alkyl is as defined above; -SO₂-(lower alkyl) wherein the lower alkyl is as defined above; -CO-(heterocycle) wherein the heterocycle is as defined above such as pyrrolidine, piperazine and morpholine, which may have 1 to 5 substituent(s) selected from the group consisting of lower alkyl and halogen, wherein the lower alkyl and halogen are as defined above, and the substitution sites are not particularly limited; and a group of the formula: -CONR^{g}R^{h} wherein R^{g} is hydrogen, lower alkyl, aryl or aralkyl and R^{h} is hydrogen, lower alkyl, aryl or aralkyl wherein the lower alkyl, aryl and aralkyl are as defined above, and the substitution sites are not particularly limited;
(10) a group of the formula: -(CH₂)ₚ-NRⁱR^{j} wherein p is an integer of 0 to 6; Rⁱ is hydrogen, acyl, lower alkyl, aryl or aralkyl and R^{j} is hydrogen, acyl, lower alkyl, aryl or aralkyl wherein the acyl, lower alkyl, aryl and aralkyl are as defined above, and the lower alkyl may have 1 to 5 substituent(s) selected from the group consisting of a group of the formula: -CONR^{k}R¹ wherein R^{k} is hydrogen, lower alkyl, aryl or aralkyl and R¹ is hydrogen, lower alkyl, aryl or aralkyl wherein the lower alkyl, aryl and aralkyl are as defined above, and the substitution sites are not particularly limited;
(11) a group of the formula:

   -CON (H or lower alkyl)-(CHR^{m})_{q}-T

   wherein q is an integer of 0 to 6; the lower alkyl is as defined above; R^{m} is hydrogen, aralkyl which is as defined above, or alkyl which is as defined above, which may be substituted by 1 to 3 substituent(s) selected from the group consisting of -OH and -CONH₂ and the substitution sites are not particularly limited; and T is hydrogen; a group of the formula: -CONRⁿR^{o} wherein Rⁿ is hydrogen, lower alkyl, aryl or aralkyl and R^{ο} is hydrogen, lower alkyl, aryl or aralkyl wherein the lower alkyl, aryl and aralkyl are as defined above; -NH-CO-R^{p} wherein R^{p} is lower alkyl which is as defined above or aralkyl which is as defined above;
   -NH-SO₂-(lower alkyl) wherein the lower alkyl is as defined above; -SO₂-(lower alkyl) wherein the lower alkyl is as defined above; -heterocycle wherein the heterocycle is as defined above, such as pyridine, pyrrolidine and morpholine, which may have 1 to 3 substituent(s) such as oxo (i.e. =O), and the substitution sites are not particularly limited; or -CO-(heterocycle) wherein the heterocycle is as defined above, such as piperidine and morpholine; and
(12) a group of the formula: -(CH₂)ᵣ-CO-NR^{t}R^{u} wherein r is an integer of 1 to 6; R^{t} is hydrogen, lower alkyl, aryl or aralkyl and R^{u} is hydrogen, lower alkyl, aryl or aralkyl wherein the lower alkyl, aryl and aralkyl are as defined above.

The substitution site on the aryl or heterocycle is any suitable position thereof, but not particularly limited. Preferable "substituent" of the above "optionally substituted thiazole" is methylsulfonylbenzyl.
The substitution sites of R² on the phenyl in Compound (I) is not particularly limited.
When Z is a group of the formula: 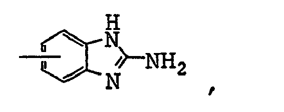 the substitution sites on the group are not particularly limited. 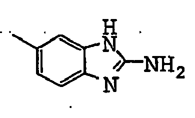 is particularly preferable.

Any nitrogen atom in the amino (i.e. -NH₂), imino (i.e. =NH or -NH-) or the like contained in Compound (I) may be protected according to the methods, which are known to those skilled in the art, such as the methods described in Protective Groups in Organic Synthesis, published by John Wiley and Sons (1980), and the like.

When Compound (I) has an asymmetric carbon atom in the structure, those skilled in the art will recognize that Compound (I) includes all stereoisomers.
Of the above-mentioned compounds, preferred are Compound (I), more preferably,
N-{4-[2-(4-{[amino(imino)methyl]amino)phenyl)ethyl]-1,3-thiazol-2-yl}acetamide (see Structure 1),
N-{4-[2-(4-{[amino(imino)methyl]amino}phenyl)ethyl]-5-[4-(methylsulfonyl)benzyl]-1,3-thia2ol-2-yl}acetamide (see Structure 46),
N-{4-[2-(4-{[hydrazino(imino)methyl]amino}phenyl)ethyl]-5-[4-(methylsulfonyl)benzyl]-1,3-thiazol-2-yl}acetamide (see Structure 48),
N-{4-[2-(4-{[hydrazino(imino)methyl]amino}phenyl)ethyl]-1,3-thiazol-2-yl)acetamide (see Structure 56), and
N-(4-{2-[4-(2-{[amino(imino)methyl]amino}ethyl)phenyl] ethyl}-1,3-thiazol-2-yl)acetamide (see Structure 107), particularly N-{4-[2-(4-{[amino(imino)methyl]amino}-phenyl)ethyl]-1,3-thiazol-2-yl}acetamide and derivatives thereof.

The term "derivative" is intended to include all compounds derived from the original compound.
The pharmaceutically acceptable salt of Compound (I) of the present invention is nontoxic and a pharmaceutically acceptable conventional salt, which is exemplified by salts with inorganic or organic base such as alkali metal salt (e.g., sodium salt, potassium salt and the like), alkaline earth metal salt (e.g., calcium salt, magnesium salt and the like), ammonium salt, and amine salt (e.g., triethylamine salt, N-benzyl-N-methylamine salt and the like).

The Compound (I) can be also formulated as a pharmaceutically acceptable acid addition salt. Examples of the pharmaceutically acceptable acid addition salts for use in the pharmaceutical composition include those derived from mineral acids, such as hydrochloric, hydrobromic, hydriodic, phosphoric, metaphosphoric, nitric and sulfuric acids, and organic acids, such as tartaric, acetic, citric, malic, lactic, fumaric, benzoic, glycolic, gluconic, succinic and arylsulfonic acids, for example, p-toluenesulfonic acid.

As a pharmaceutically acceptable salt of Compound (I) represented by the formula (I), a pharmaceutically acceptable acid addition salt such as (mono-, di- or tri-) hydrochloride and hydriodide, particuraly hydrochloride, is preferable.

The above-mentioned Compound (I) may be commercially available or can be produced based on a known reference. The composition can be administered in accordance with the present inventive method by any suitable route. Suitable routes of administration include systemic, such as orally or by injection, topical, periocular (e.g., subTenon's), subconjunctival, intraocular, intravitreal, intracameral, subretinal, suprachoroidal and retrobulbar administrations. The manner in which the VAP-1 inhibitor is administered is dependent, in part, upon whether the treatment of a VAP-1 associated disease is prophylactic or therapeutic.

The composition is preferably administered as soon as possible after it has been determined that a subject such as mammal, specifically a human, is at risk for a VAP-1 associated disease (prophylactic treatments) or has begun to develop a VAP-1 associated disease (therapeutic treatments). Treatment will depend, in part, upon the particular VAP-1 inhibitor to be used, the amount of the VAP-1 inhibitor to be administered, the route of administration, and the cause and extent, if any, of a VAP-1 associated disease realized.

One skilled in the art will appreciate that suitable methods of administering a VAP-1 inhibitor, which is useful in the present inventive method, are available. Although more than one route can be used to administer a particular VAP-1 inhibitor, a particular route can provide a more immediate and more effective reaction than another route. Accordingly, the described routes of administration are merely exemplary and are in no way limiting.

The dose of the composition administered to the administration subject such as animal including human, particularly a human, in accordance with the present invention should be sufficient to effectuate the desired response in the subject over a reasonable time frame. One skilled in the art will recognize that dosage will depend upon a variety of factors, including the strength of the particular VAP-1 inhibitor to be employed, the age, species, conditions or disease states, and body weight of the subject, as well as the degree of a VAP-1 associated disease. The size of the dose also will be determined by the route, timing and frequency of administration as well as the existence, nature, and extent of any adverse side effects that might accompany the administration of a particular VAP-1 inhibitor and the desired physiological effect. It will be appreciated by one of ordinary skill in the art that various conditions or disease states may require prolonged treatment involving multiple administrations.

Suitable doses and dosage regimens can be determined by conventional range-finding techniques known to those of ordinary skill in the art. Generally, treatment is initiated with smaller dosages, which are less than the optimum dose of the compound. Thereafter, the dosage is increased by small increments until the optimum effect under the circumstances is reached.

Generally, the compound (I) can be administered in the dose of from about 1 µg/kg/day to about 300 mg/kg/day, preferably from about 0.1 mg/kg/day to about 10 mg/kg/day, which is given in a single dose or 2 to 4 doses a day or in a sustained manner.

In the present specification and claims, the "aqueous composition" means clear aqueous solution. The aqueous composition of the present invention may be provided as ophthalmic solution, nasal solution, ear solution, inhalant solution, spray, oral solution, injectable solution for intravenous, intraarterial, subcutaneous, intramuscular, interperitoneal or intraocular administration. Since the additives comprised in the aqueous composition of the present invention, i.e. additives selected from the group consisting of polyol, sugar other than glucose, sugar alcohol, boric acid or its salt will not affect on solubility of Compound (1), a stable aqueous dosage' form with a long shelf life can be provided.

According to the invention, the additives are those known to be useful for adjusting osmotic pressure of aqueous solution. Examples of the additives used in the present invention may include polyols such as glycerin, polyethylene glycol, propylene glycols and polyvinyl alcohol; saccharides or sugar alcohols such as sorbitol, mannitol and xylitol; boric acid and its salt. Those additives may be used either solely or in combination two or more of them depending on the desired formula. Especially preferable additives include glycerin, mannitol, boric acid or its salt.

The concentration of the additives in the aqueous composition of the present invention may be determined so that the osmotic pressure of the composition is adjusted appropriately. The art can determine the concentration based on the kind of the thiazol derivative, the amount of the thiazole derivative as well as the kind and molecular weight of the additives. Typically, the amount of the additive may be 0.001-10w/v%, preferably 0.01-5 w/v% based on total volume of the composition.

The aqueous composition of the present invention may further comprises other additives which are generally used in manufacturing medical compositions, such as a buffeting agent, a preservative, a stabilizer and a thickening-agent so long as those additive affect on the solubulity of the thiazole derivative or Compound 1 of the present invention. Examples of said additives may include preservatives such as benzalkonium chloride, chlorobutanol and paraoxybenzoates; thickening agent such as povidone and methylcellulose.

The aqueous composition of the present invention may further comprise or may be co-administrated with a pharmaceutically active compound other than Compound (I). By "co-administration" is meant administration before, concurrently with, e.g., in combination with the VAP-1 inhibitor or Compound (I) thiazole derivative in the same formulation or in separate formulations, or after administration of the VAP-1 inhibitor as described above. For example, corticosteroids, prednisone, methylprednisolone, dexamethasone, or triamcinolone acetinide, or noncorticosteroid anti-inflammatory compounds, such as ibuprofen or flubiprofen, can be co-administered. Similarly, vitamins and minerals, e.g., zinc, anti-oxidants, e.g., carotenoids (such as a xanthophyll carotenoid like zeaxanthin or lutein), and micronutrients can be co-formulated or co-administered.

In addition, the aqueous composition according to the present invention is also useful for preparing a medicament such as a therapeutic or prophylactic agent for the VAP-1 associated diseases.

Examples of the preferred compound (I) thiazole derivative according to the present invention are listed in the following tables.

| **No.** | **Structure** | **No.** | **Structure** |
|---|---|---|---|
| 1 | 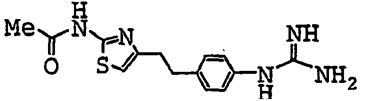 | 11 | 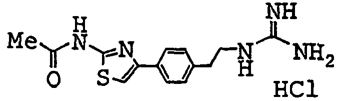 |
| 2 | 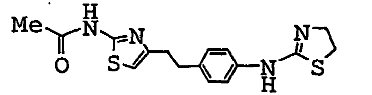 | 12 | 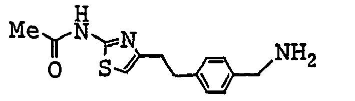 |
| 3 | 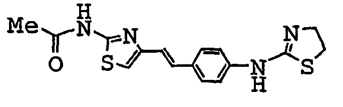 | 13 | 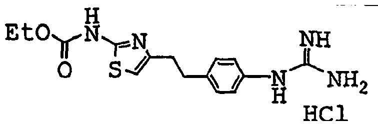 |
| 4 | 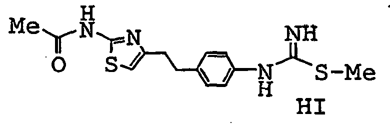 | 14 | 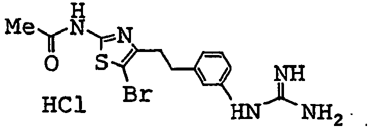 |
| 5 | 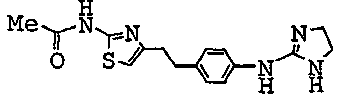 | 15 | 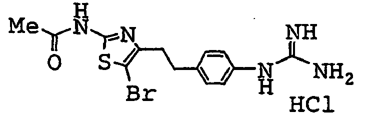 |
| 6 | 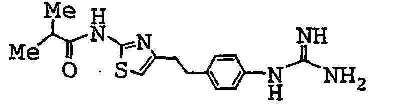 | 16 | 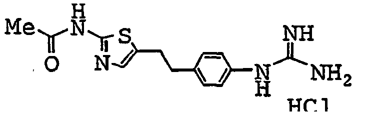 |
| 7 | 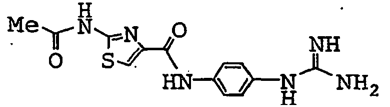 | 17 | 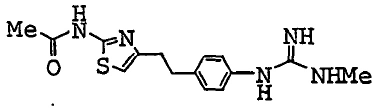 |
| 8 | 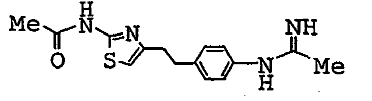 | 18 | 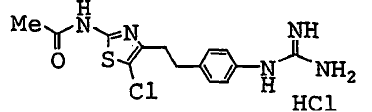 |
| 9 | 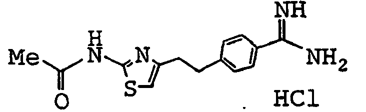 | 19 | 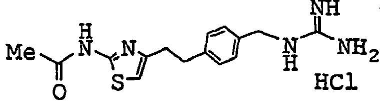 |
| 10 | 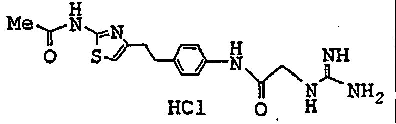 | 20 | 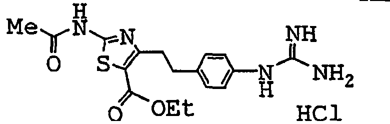 |
| 21 | 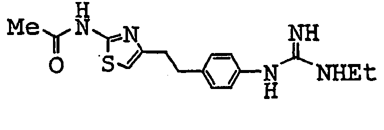 | 26 | 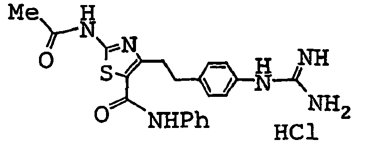 |
| 22 | 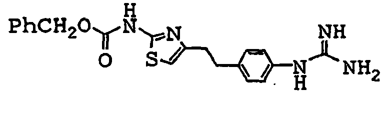 | 27 | 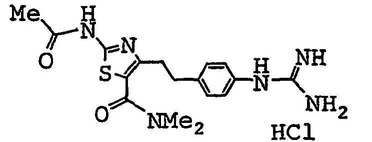 |
| 23 | 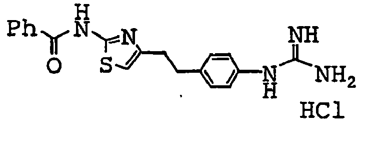 | 28 | 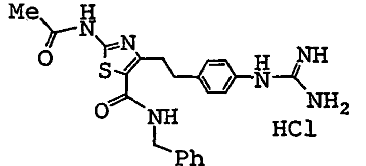 |
| 24 | 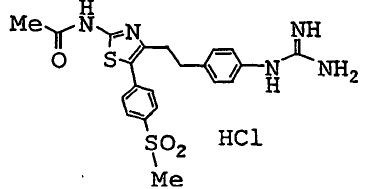 | 29 | 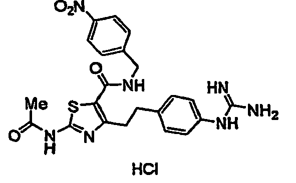 |
| 25 | 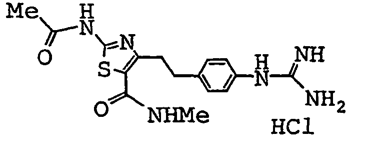 | 30 | 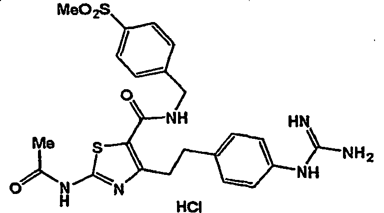 |
| 31 | 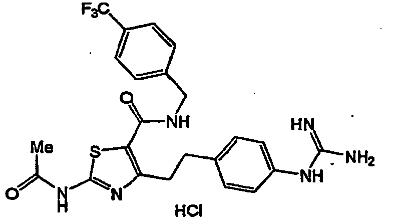 | 36 | 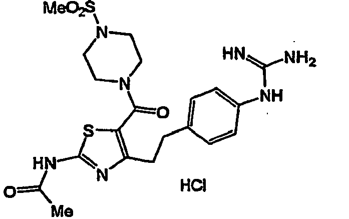 |
| 32 | 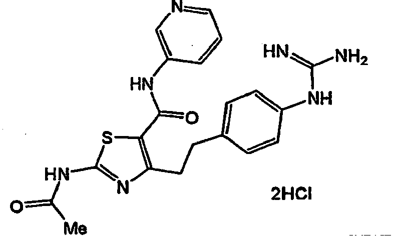 | 37 | 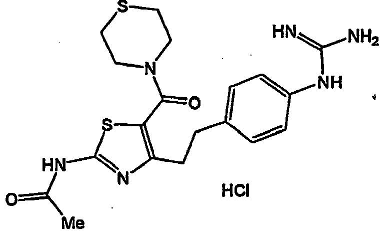 |
| 33 | 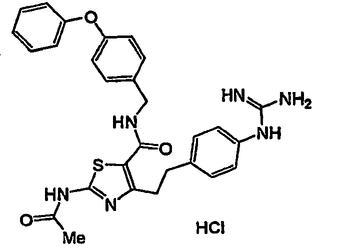 | 38 | 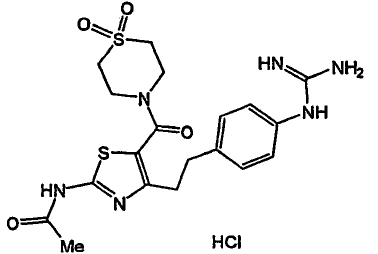 |
| 34 | 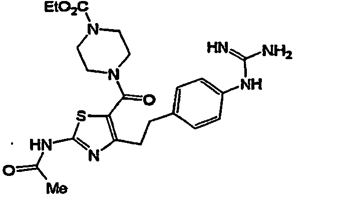 | 39 | 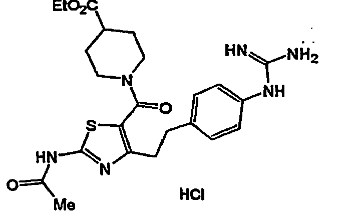 |
| 35 | 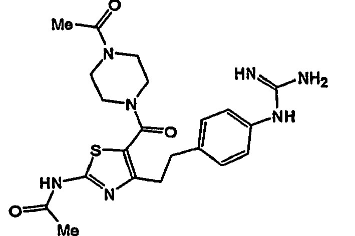 | 40 | 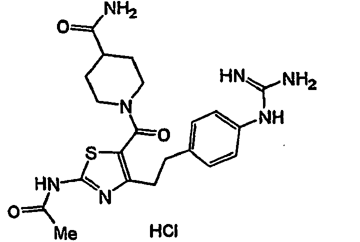 |
| 41 | 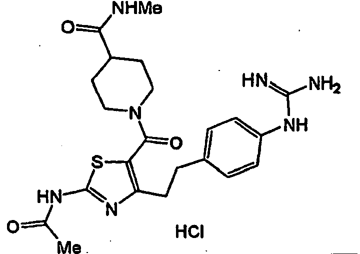 | 46 | 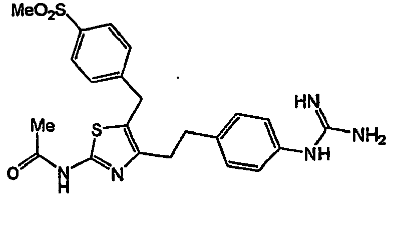 |
| 42 | 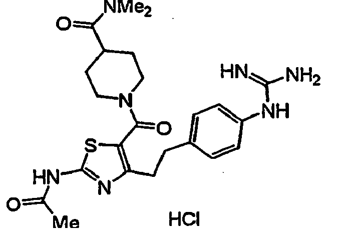 | 47 | 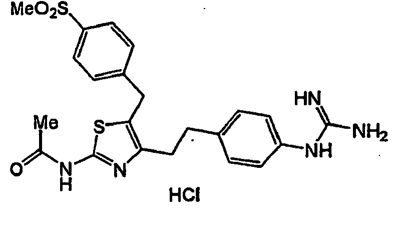 |
| 43 | 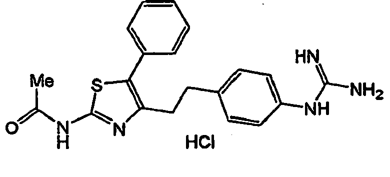 | 48 | 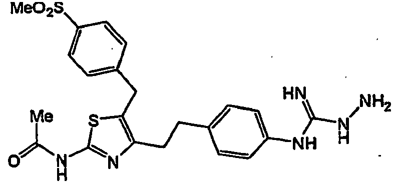 |
| 44 | 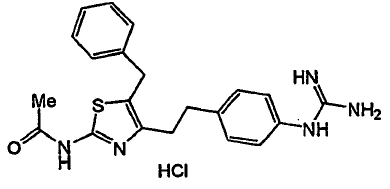 | 49 | 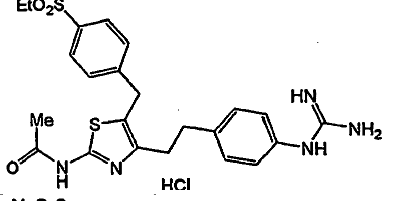 |
| 45 | 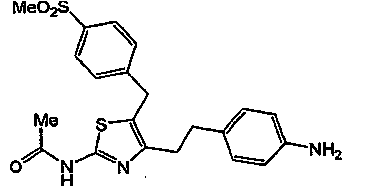 | 50 | 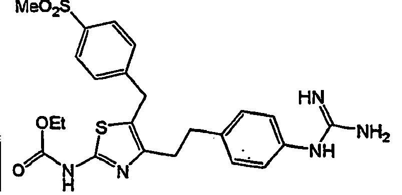 |
| 51 | 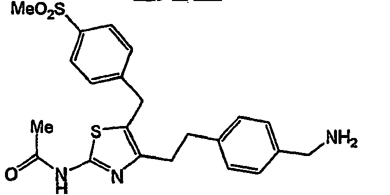 | 56 | 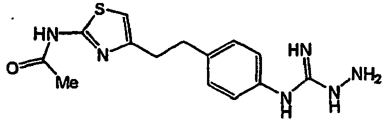 |
| 52 | 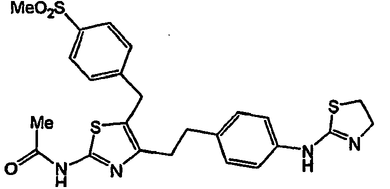 | 57 | 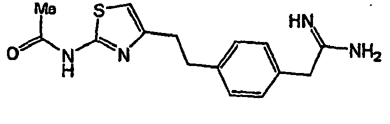 |
| 53 | 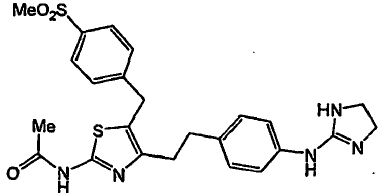 | 58 | 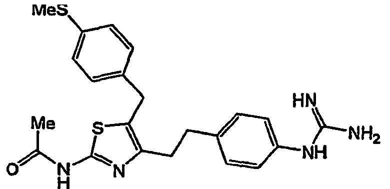 |
| 54 | 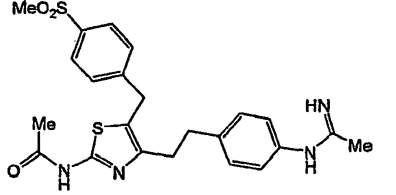 | 59 | 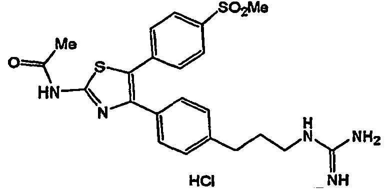 |
| 55 | 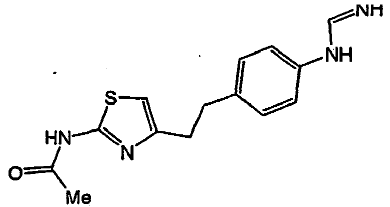 | 60 | 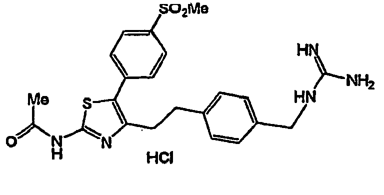 |
| 61 | 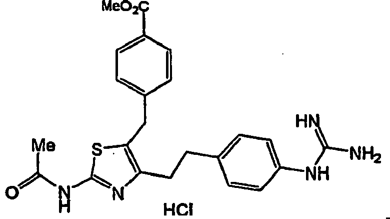 | 66 | 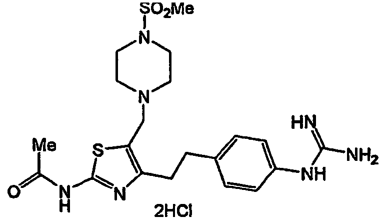 |
| 62 | 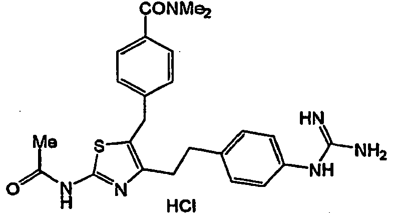 | 67 | 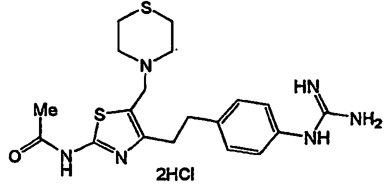 |
| 63 | 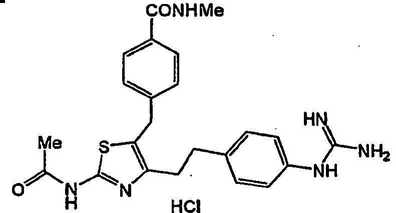 | 68 | 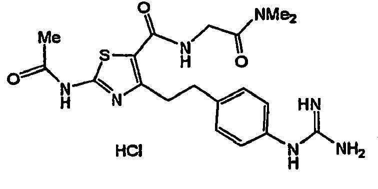 |
| 64 | 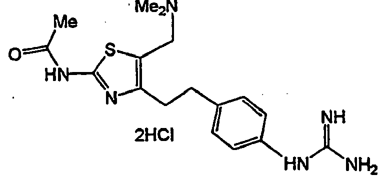 | 69 | 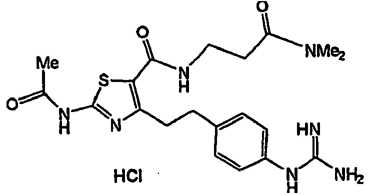 |
| 65 | 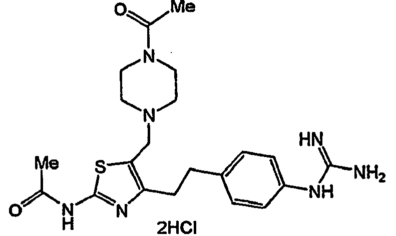 | 70 | 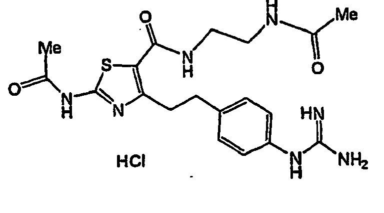 |
| 71 | 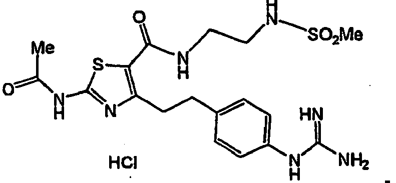 | 76 | 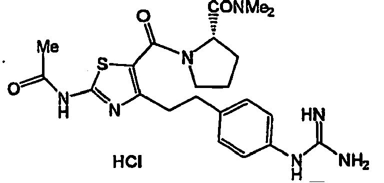 |
| 72 | 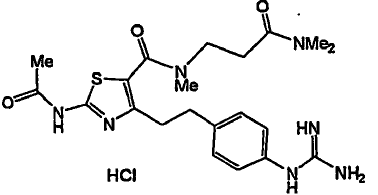 | 77 | 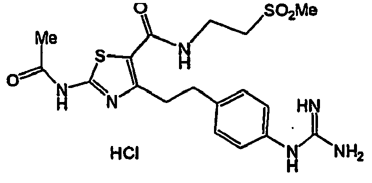 |
| 73 | 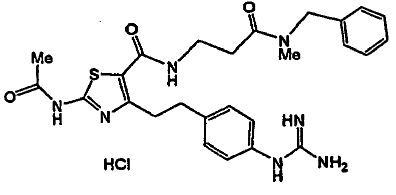 | 78 | 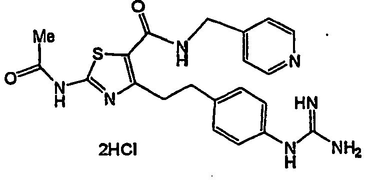 |
| 74 | 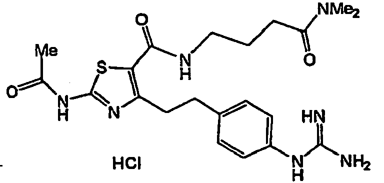 | 79 | 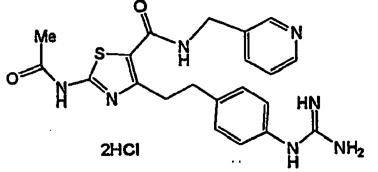 |
| 75 | 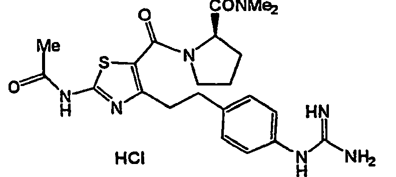 | 80 | 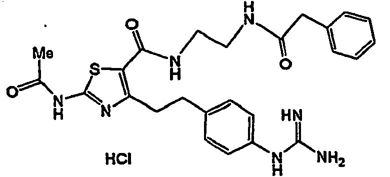 |
| 81 | 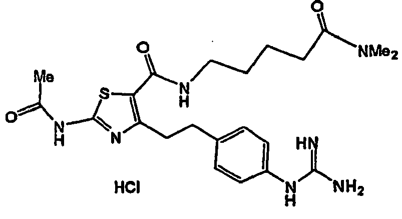 | 86 | 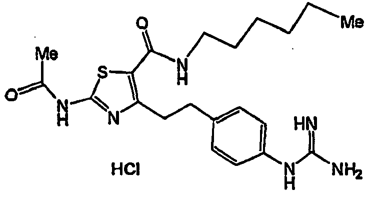 |
| 82 | 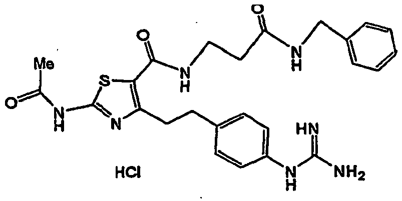 | 87 | 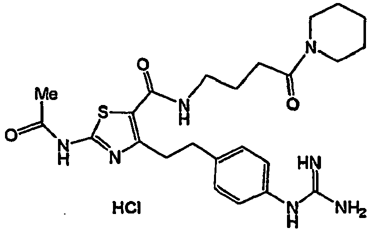 |
| 83 | 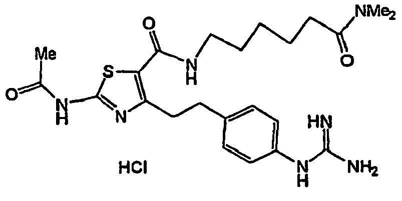 | 88 | 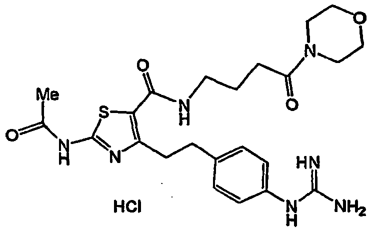 |
| 84 | 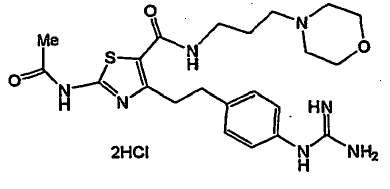 | 89 | 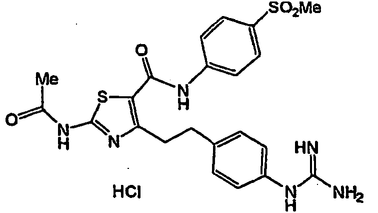 |
| 85 | 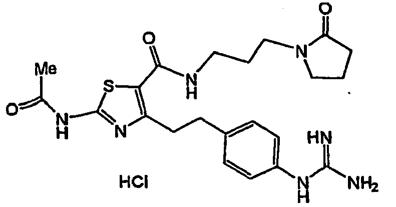 | 90 | 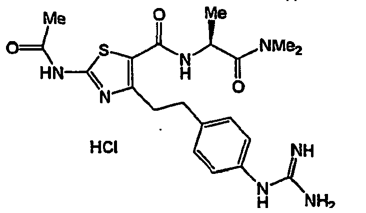 |
| 91 | 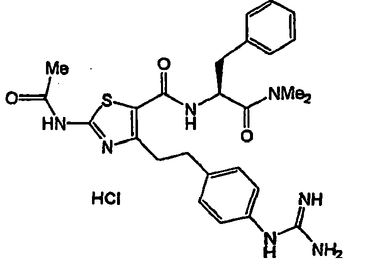 | 96 | 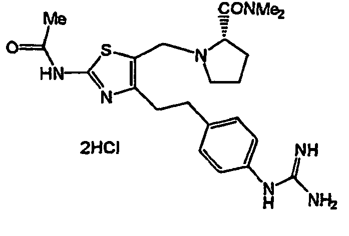 |
| 92 | 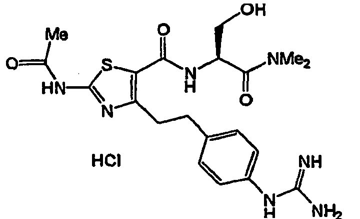 | 97 | 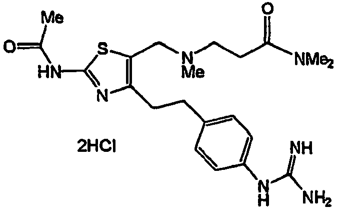 |
| 93 | 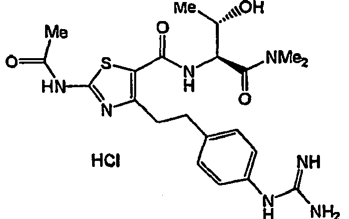 | 98 | 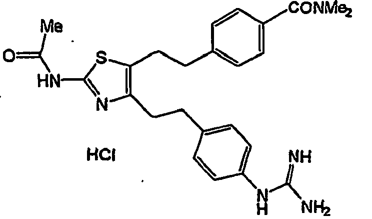 |
| 94 | 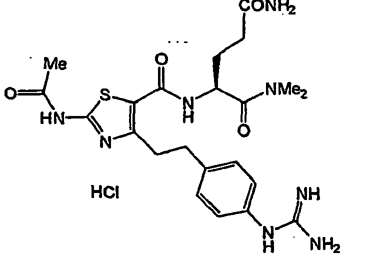 | 99 | 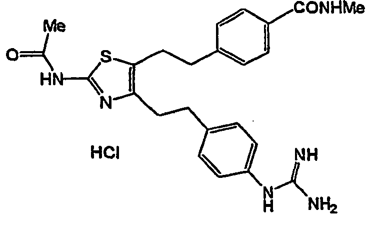 |
| 95 | 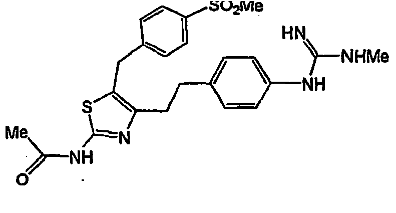 | 100 | 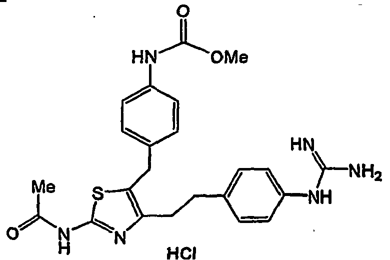 |
| 101 | 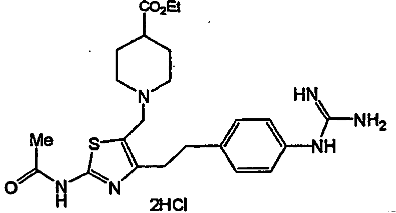 | 106 | 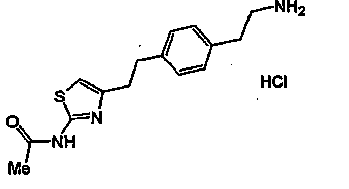 |
| 102 | 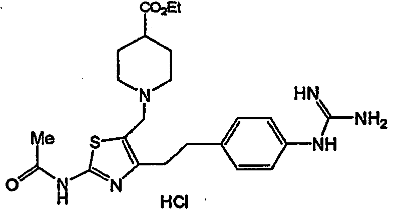 | 107 | 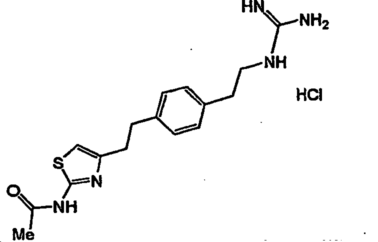 |
| 103 | 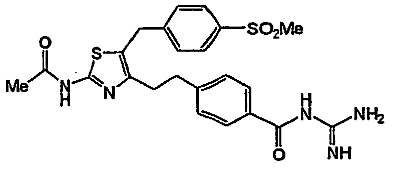 | 108 | 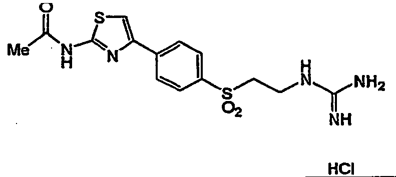 |
| 104 | 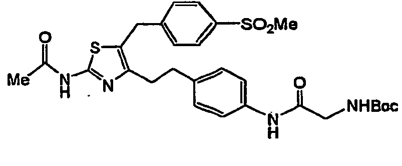 | 109 | 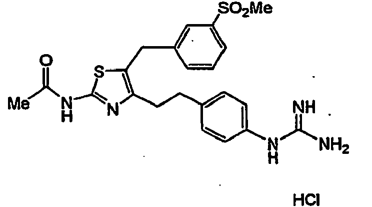 |
| 105 | 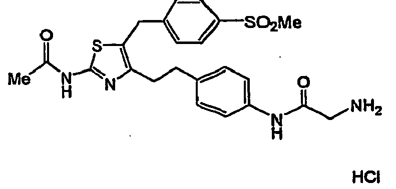 | 110 | 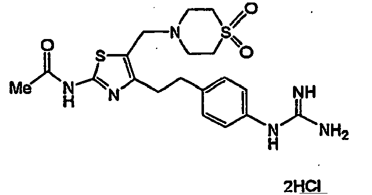 |
| 111 | 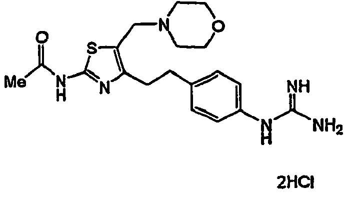 | 116 | 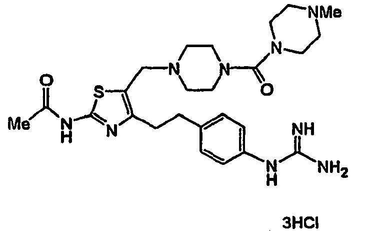 |
| 112 | 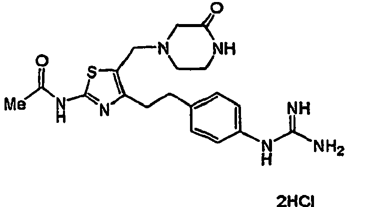 | 117 | 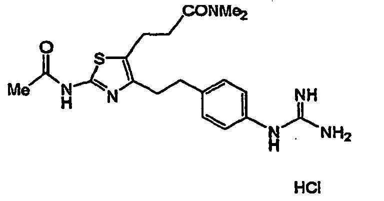 |
| 113 | 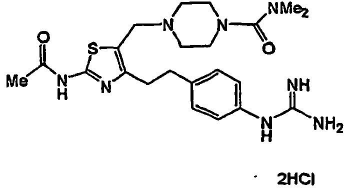 | 118 | 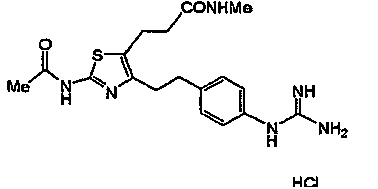 |
| 114 | 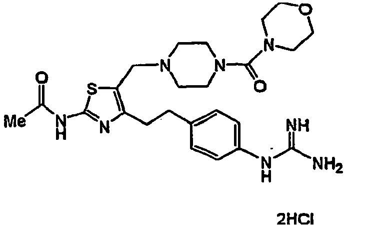 | 119 | 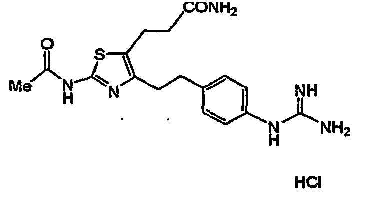 |
| 115 | 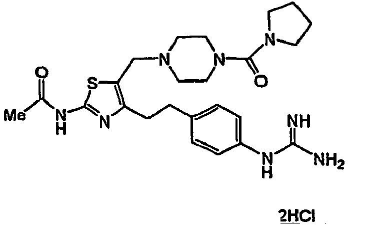 | 120 | 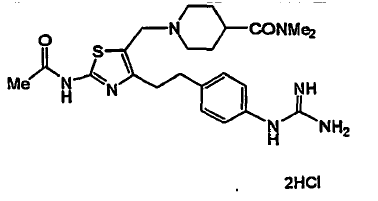 |
| 121 | 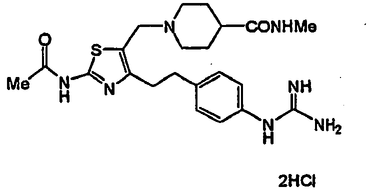 | 126 | 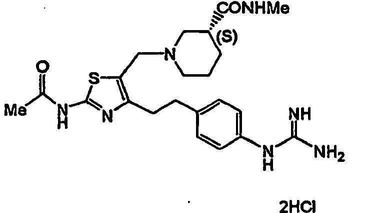 |
| 122 | 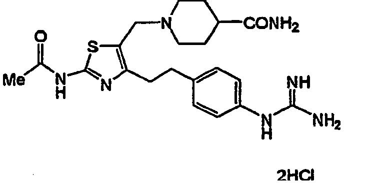 | 127 | 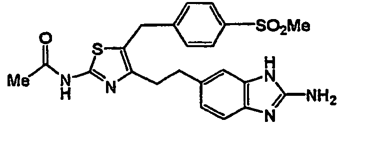 |
| 123 | 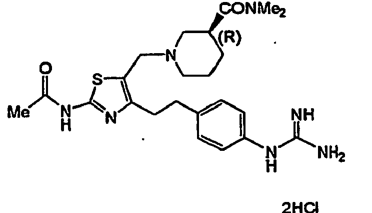 | 128 | 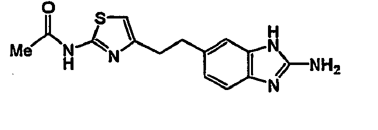 |
| 124 | 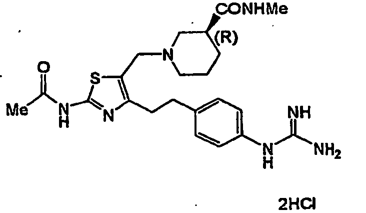 | 129 | 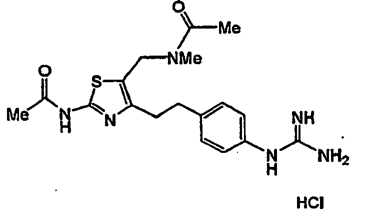 |
| 125 | 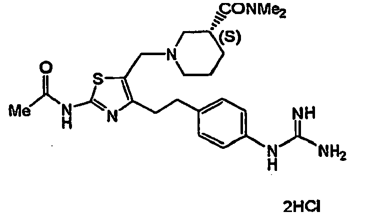 | 130 | 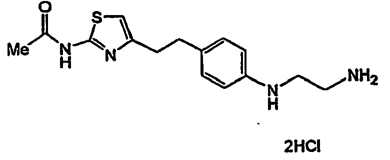 |
| 131 | 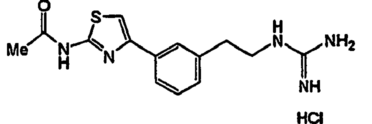 | | |
| 132 | 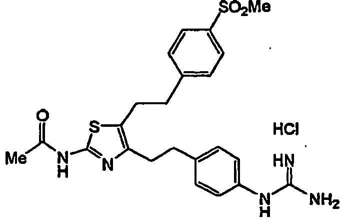 | | |

The present invention is further illustrated by means of the examples shown below. The examples, however, should not be used for limiting the scope of the invention in any means.

### Test Example 1

### Compound A:

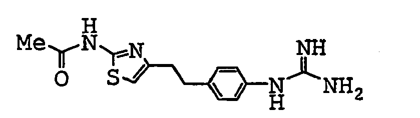

### Compound B:

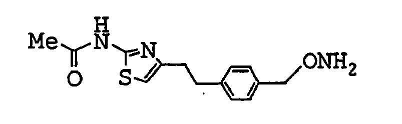

### Compound C:

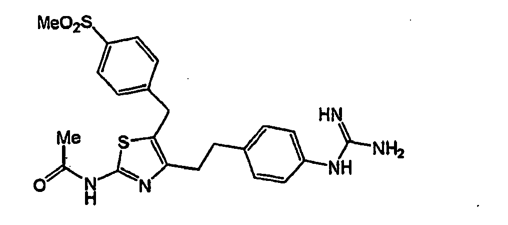

Compound A was added with distilled water (injection grade) and stirred to give an aqueous saturated solution of Compound A (3.55 mg/mL, about pH 7).

Compound B was added with distilled water (injection grade) and stirred with adding hydrochloric acid to give an aqueous saturated solution of Compound B (1.12 mg/mL, about pH 3).

Compound C was added with distilled water (injectable grade) and stirred with adding hydrochloric acid to give an aqueous saturated solution of Compound C (10.73 mg/mL, about pH 7).

Sodium chloride was added to thus obtained aqueous saturated solution of Compound A, B, and C respectively in an amount to give 0.4% NaCl solution and the mixture was stirred. Thus obtained mixture was observed for precipitation and the solubility, i.e. the concentration of the Compound A-C in the solution or supernatant (mg/mL).

Similarly, the saturated aqueous solution of Compound A-C was added with sodium chloride in an amount to give 0.85% solution, and precipitation and solubility were observed.

Results are shown in table 1 below.

**TABLE 1 effect of sodium chloride on the solubility of the Compound A, B and C**

| | conc. of NaCl (%) | Precipitation | Solubility (mg/mL) | % Solubulity |
|---|---|---|---|---|
| A | 0 | no | 3.55 | =100 |
| | 0.4 | yes | 1.11 | 31 |
| | 0.85 | yes | 0.69 | 20 |
| B | 0 | no | 1.12 | =100 |
| | 0.4 | no | 1.17 | 100 |
| | 0.85 | no | 1.19 | 100 |
| C | 0 | no | 10.73 | =100 |
| | 0.4 | yes | 1.76 | 16 |
| | 0.85 | yes | 0.86 | 8 |

### Test Example 2

To the saturated solution of Compound C prepared in the same manner as test example 1, glycerin in an amount to give 2.5% solution was added and the mixture was stirred. Similarly, mannitol in an amount to give 3.5% solution, boric acid in an amount to give 2% solution, potassium chloride in an amount to give 0.2% solution, disodium hydrogenphosphate in an amount to give 1% and sodium citrate in an amount to give 1% solution were added to the aqueous saturated solution of Compound C respectively. Precipitation and solubility, i.e. the change of the concentration of Compound C in the solution or supernatant were observed.

**Table 2: Effect of the additives on solubility of Compound C.**

| additives, conc. in the solution (%) | | precipitation | Conc. of C w/o additive (mg/mL) (A) | Conc. of C/w additive (mg/mL) (B) | B/A ratio (%) |
|---|---|---|---|---|---|
| Glycerin | 2.5% | no | 11.51 | 10.91 | 95 |
| Mannitol | 3.5% | no | 11.51 | 11.05 | 96 |
| Boric acid | 2% | no | 11.51 | 11.59 | 100 |
| Potassium Chloride | 0.2% | yes | 10.73 | 3.16 | 29 |
| disodium hydrogenphosphate | 1% | yes | 10.73 | 2.54 | 24 |
| sodium citrate | 1% | yes | 10.73 | 0.48 | 4 |

### Test Example 3

Compound C was added with glycerin and distilled water and stirred with adding hydrochloric acid to give an aqueous solution (Conc. of Compound C: 0.3%, conc. of glycerin: 2.5%, about pH 6). Similarly, aqueous solutions comprising Compound C and the additives shown in the table 3 were prepared.

Thus obtained solution was stored at 40°C in the LDPE container and the concentration of Compound C in the solution was observed over time. Result is shown Table 3 below:

**Table 3. Effect of the additives on the stability of the aqueous solution comprising Compound C**

| Additives, conc. in the solution (%) | | Change in Concentration of Compound C (vs the initial conc.) (%) | | | |
|---|---|---|---|---|---|
| | | Initial | 40°C | | |
| | | | 1 month | 3 months | 6 months |
| Glycerin | 2.5% | =100 | 105.4 | 110.6 | 112.3 |
| Mannitol | 4.7% | =100 | 103.7 | 108.4 | 109.6 |
| Boric acid borax | 1.68% 0.018% | =100 | 104.9 | 106.2 | 108.2 |

### Test Example 4

### Compound D

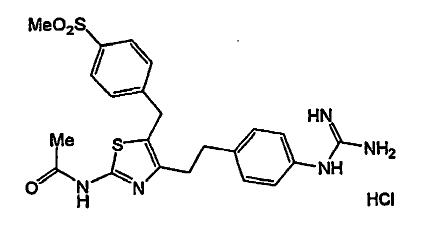

Compound D was added with glycerin and distilled water and stirred to give clear aqueous solution (Conc. of Compound D: 0.3%, conc. of glycerin: 2.5%, about pH 6). Similarly, aqueous solutions comprising the same concentration of Compound D and the additives shown in table 4 were prepared.

Thus obtained solution was stored at 40°C in the LDPE container and concentration of Compound D in the solution was observed over time. Result is shown Table 4 below:

**Table 4. Effect of the additives on the stability of the aqueous solution comprising Compound D**

| Additives, conc. in the solution (%) | | Change in Concentration of Compound D (vs the initial conc.) (%) | | | |
|---|---|---|---|---|---|
| | | initial | 40°C | | |
| | | | 1 month | 3 months | 6 months |
| Glycerin | 2.5% | =100 | 100.8 | 107.7 | 104.4 |
| Boric acid borax | 1.68% 0.018% | =100 | 99.7 | 105.8 | 102.6 |

### INDUSTRIAL APPLICABILITY

The present invention provides an aqueous composition comprising a thiazole derivative of the formula (I): R¹-NH -X-Y-Z (I)
wherein each symbol is as defined above, or a pharmaceutically acceptable salt thereof, and an additive selected from the group consisting of polyol, sugar other than glucose, sugar alcohol, boric acid or its salt, and water.

The aqueous composition of the present invention is very stable and therefore, aqueous dosage formulae which can be stably stored for a long time can be provided.

## Claims

1. An aqueous composition comprising a thiazole derivative of the formula (I):
R¹-NH-X-Y-Z (I)
wherein
R¹ is acyl;
X is a bivalent residue derived from optionally substituted thiazole;
Y is a bond, lower alkylene, lower alkenylene or -CONH-; and
Z is a group of the formula: 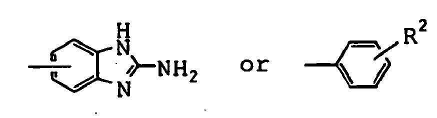 wherein R² is a group of the formula: -A-B-D-E
wherein A is a bond, lower alkylene, -NH- or -SO₂-;
B is a bond, lower alkylene, -CO- or -O-;
D is a bond, lower alkylene, -NH- or -CH₂NH-, provided that when B is -CO- or -O-, D is not a bond; and
E is optionally protected amino, -N=CH₂, 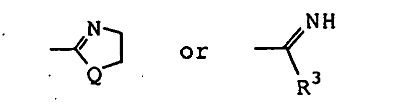 wherein
Q is -S- or -NH-; and
R³ is hydrogen, lower alkyl, lower alkylthio or -NH-R⁴ wherein R⁴ is hydrogen, -NH₂ or lower alkyl;
or a pharmaceutically acceptable salt thereof, water and an additive selected from the group consisting of polyol, sugar other than glucose, sugar alcohol, boric acid or its salt.

2. The composition of claim 1, wherein Z of the formula (I) is a group of the formula: 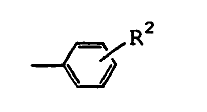 wherein R² is a group of the formula: 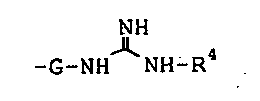 (wherein G is a bond, -NHCOCH₂- or lower alkylene and R⁴ is hydrogen, -NH₂ or lower alkyl) ; -NH₂; -CH₂NH₂; -CH₂ONH₂; -CH₂ON=CH₂; 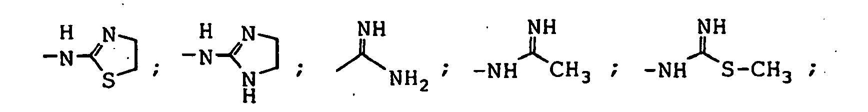 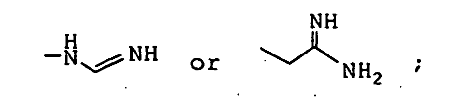 or a pharmaceutically acceptable salt thereof.

3. The composition of claim 2, wherein R² of the formula (I) is a group of the formula: 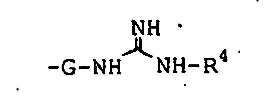 (wherein G is a bond, -NHCOCH₂- or lower alkylene and R⁴ is hydrogen or lower alkyl) ; -CH₂NH₂; -CH₂ONH₂; -CH₂ON=CH₂; 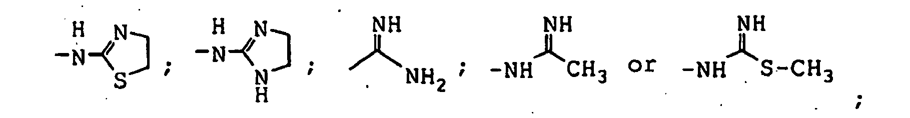 or a pharmaceutically acceptable salt thereof.

4. The composition of any of claims 1 to 3, wherein R¹ of the formula (I) is alkylcarbonyl and X is a bivalent residue derived from thiazole optionally substituted by methylsulfonylbenzyl, or a pharmaceutically acceptable salt thereof.

5. The composition of claim 1, wherein the tiazole derivative is
N-{4-[2-(4-{[amino(imino)methyl]amino}phenyl)ethyl]-1,3-thiazol-2-yl}acetamide,
N-{4-(2-(4-{[amino(imino)methyl]amino}phenyl)ethyl]-5-[4-(methylsulfonyl)benzyl]-1,3-thiazol-2-yl)acetamide,
N-{4-[2-(4-{[hydrazino(imino)methyl]amino}phenyl)ethyl]-5-[4-(methylsulfonyl)benzyl]-1,3-thiazol-2-yl)acetamide,
N-{4-[2-(4-{[hydrazino(imino)methyl]amino}phenyl)ethyl]-1,3-thiazol-2-yl}acetamide, or
N-(4-{2-[4-(2-{[amino(imino)methyl]amino}ethyl)phenyl]ethyl}-1,3-thiazol-2-yl)acetamide,
or a pharmaceutically acceptable salt thereof.

6. The composition of any one of claims 1 to 5, wherein the polyol is glycerin.

7. The composition of any one of claims 1 to 5, wherein the sugar alcohol is mannitol.

## Patentansprüche

1. Wässrige Zusammensetzung, umfassend ein Thiazolderivat der Formel (I):
R¹-NH-X-Y-Z (I)
worin
R¹ Acyl ist;
X ein bivalenter Rest ist, der von einem gegebenenfalls substituierten Thiazol abgeleitet ist;
Y eine Bindung, Niederalkylen, Niederalkenylen oder -CONHist und
Z eine Gruppe der Formel: 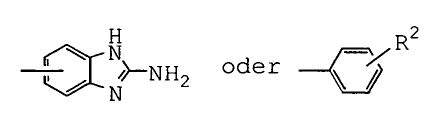 ist,
worin R² eine Gruppe der Formel: -A-B-D-E ist,
worin A eine Bindung, Niederalkylen, -NH- oder -SO₂- ist;
B eine Bindung, Niederalkylen, -CO- oder -O- ist;
D eine Bindung, Niederalkylen, -NH- oder -CH₂NH- ist, mit der Maßgabe, dass, wenn B -CO- oder -O- ist, D keine Bindung ist, und
E gegebenenfalls geschütztes Amino, -N=CH₂, 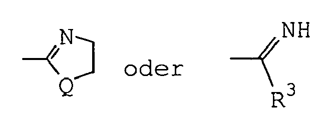 ist,
worin
Q -S- oder -NH- ist und
R³ Wasserstoff, Niederalkyl, Niederalkylthio oder NH-R⁴, worin R⁴ Wasserstoff, NH₂ oder Niederalkyl ist, ist;
oder ein pharmazeutisch verträgliches Salz davon, Wasser und ein Additiv, ausgewählt aus der Gruppe, bestehend aus Polyol, anderem Zucker als Glucose, Zuckeralkohol, Borsäure oder ihrem Salz.

2. Zusammensetzung gemäß Anspruch 1, wobei Z der Formel (I) eine Gruppe der Formel 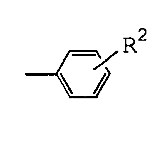 ist,
worin R² eine Gruppe der Formel: 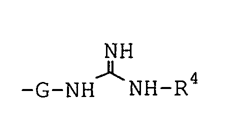 (worin G eine Bindung, -NHCOCH₂- oder Niederalkylen ist und R⁴ Wasserstoff, -NH₂ oder Niederalkyl ist); -NH₂; -CH₂NH₂; -CH₂ONH₂; -CH₂ON=CH₂; 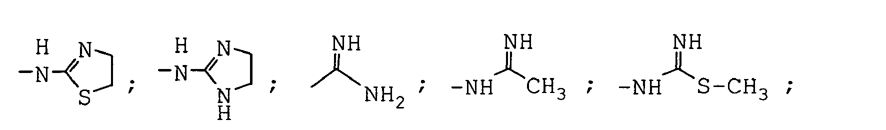 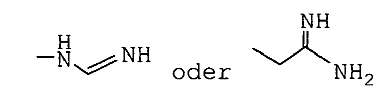 ist;
oder ein pharmazeutisch verträgliches Salz davon.

3. Zusammensetzung gemäß Anspruch 2, wobei R² der Formel (I) eine Gruppe der Formel: 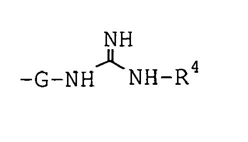 (worin G eine Bindung, -NHCOCH₂- oder Niederalkylen ist und R⁴ Wasserstoff oder Niederalkyl ist); -CH₂NH₂; -CH₂ONH₂; -CH₂ON=CH₂; 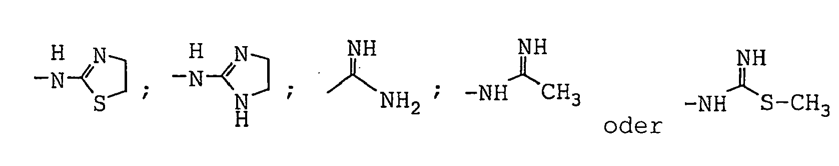 ist;
oder ein pharmazeutisch verträgliches Salz davon.

4. Zusammensetzung gemäß einem der Ansprüche 1 bis 3, wobei R¹ der Formel (I) Alkylcarbonyl ist und X ein bivalenter Rest ist, der von Thiazol, gegebenenfalls substituiert mit Methylsulfonylbenzyl, abgeleitet ist, ist oder ein pharmazeutisch verträgliches Salz davon.

5. Zusammensetzung gemäß Anspruch 1, wobei das Thiazolderivat ist
N-{4-[2-(4-{[Amino(imino)methyl]amino}phenyl)ethyl]-1,3-thiazol-2-yl}acetamid,
N-{4-[2-(4-{[Amino(imino)methyl]amino}phenyl)ethyl]-5-[4-(methylsulfonyl)benzyl]-1,3-thiazol-2-yl}acetamid,
N-{4-[2-(4-{[Hydrazino(imino)methyl]amino}phenyl)ethyl]-5-[4-(methylsulfonyl)benzyl]-1,3-thiazol-2-yl}acetamid,
N-{4-[2-(4-{[Hydrazino(imino)methyl]amino}phenyl)ethyl]-1,3-thiazol-2-yl}acetamid, oder
N-(4-{2-[4-(2-{[Amino(imino)methyl]amino}ethyl)phenyl]ethyl}-1,3-thiazol-2-yl)acetamid,
oder ein pharmazeutisch verträgliches Salz davon.

6. Zusammensetzung gemäß einem der Ansprüche 1 bis 5, wobei das Polyol Glycerin ist.

7. Zusammensetzung gemäß einem der Ansprüche 1 bis 5, wobei der Zuckeralkohol Mannit ist.

## Revendications

1. Composition aqueuse comprenant un dérivé de thiazole représenté par la formule (I):
R¹-NH-X-Y-Z (I)
dans laquelle
R¹ représente un groupe acyle;
X représente un radical divalent dérivé de thiazole étant le cas échéant substitué;
Y représente une liaison, un groupe alcoylène inférieur, un groupe alcénylène inférieur ou -CONH-; et
Z représente un groupe de formule: 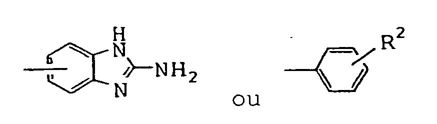 où R² représente un groupe de formule:
-A-B-D-E
dans laquelle A représente une liaison, un groupe alcoylène inférieur, un groupe -NH-, ou un groupe -SO₂-;
B représente une liaison, un groupe alcoylène inférieur, -CO- ou -O-;
D représente une liaison, un groupe alcoylène inférieur, -NH- ou -CH₂NH-, sous réserve que lorsque B représente -CO- ou -O-, D n'est pas une liaison; et
E représente un groupe amino étant le cas échéant substitué, -N=CH₂, 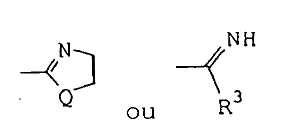 où
Q représente -S-ou -NH- et
R³ représente hydrogène, un groupe alkyle inférieur, un groupe thioalkyle ou -NH-R⁴ où R⁴ représente hydrogène, -NH₂ ou un groupe alkyle inférieur;
ou un sel pharmaceutiquement acceptable de celui-ci, de l'eau et un additif choisi parmi le groupe consistant en polyol, un sucre autre que le glucose, alditol, acide borique ou sel de celui-ci.

2. Composition selon la revendication 1, dans laquelle Z de la formule (I) est un groupe de formule: 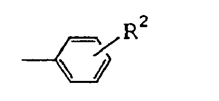 dans laquelle R² est un groupe de formule: 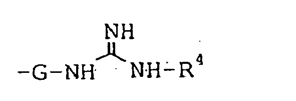 (dans laquelle G est une liaison, -NHCOCH₂- ou un groupe alcoylène inférieur et R⁴ est hydrogène, -NH₂ ou un groupe alkyle inférieur); -NH₂; -CH₂NH₂; -CH₂ONH₂; -CH₂ON=CH₂; 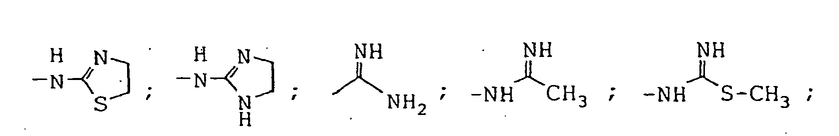 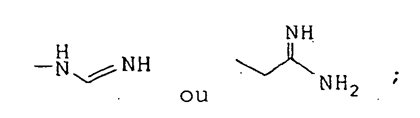 ou un sel pharmaceutiquement acceptable de celui-ci.

3. Composition selon la revendication 2, dans laquelle R² de formule (I) est un groupe de formule: 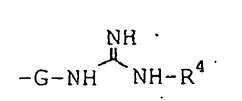 (dans laquelle G est une liaison, -NHCOCH₂- ou un groupe alcoylène inférieur est R⁴ est hydrogène ou un groupe alkyle inférieur); -CH₂NH₂; -CH₂ONH₂; -CH₂ON=CH₂; 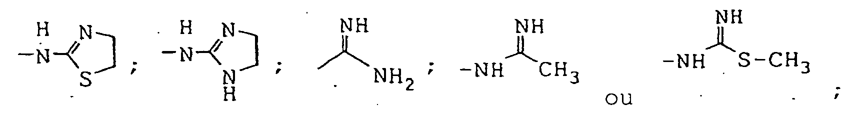 ou un sel pharmaceutiquement acceptable de celui-ci.

4. Composition selon l'une quelconque des revendications 1 à 3, dans laquelle R¹ de la formule (I) est un groupe alkylcarbonyle et X est un radical divalent dérivé de thiazole étant le cas échéant substitué par méthylsulfonylbenzyle, ou un sel pharmaceutiquement acceptable de celui-ci.

5. Composition selon la revendication 1, dans laquelle le dérivé de thiazole est
le N-{4-[2-(4-{[amino(imino)méthyl]amino}phényl)éthyl]-1,3-thiazol-2-yl}acétamide,
le N-{4-[2-(4-{[amino(imino)méthyl]amino}phényl)éthyl]-5-[4-(méthylsulfonyl)benzyle]-1,3-thiazol-2-yl}acétamide,
le N-{4-[2-(4-{[hydrazino(imino)méthyl]amino}phényl)éthyl]-5-[4-(méthylsulfonyl)benzyl]-1,3-thiazol-2-yllacétamide,
le N-{4-[2-(4-{[hydrazino(imino)méthyl]amino}phényl)éthyl]-1,3-thiazol-2-yl}acétamide ou
le N-(4-{2-[4-(2-{[amino(imino)méthyl]amino}éthyl)phenyl]éthyl}-1,3-thiazol-2-yl)acétamide
ou un sel pharmaceutiquement acceptable de celui-ci.

6. Composition selon l'une quelconque des revendications 1 à 5, dans laquelle le polyol est le glycérol.

7. Composition selon l'une quelconque des revendications 1 à 5, dans laquelle l'alditol est le mannitol.
